# EUROPEAN PATENT SPECIFICATION

(11) **EP 2 227 557 B1**
(45) Date of publication and mention of the grant of the patent: **12.12.2012**
(21) Application number: 08853161.1
(22) Date of filing: 27.11.2008
(51) Int. Cl.: C12Q 1/68

(54) **MARKER ASSISTED DIRECTED EVOLUTION**
MARKER-UNTERSTÜTZTE GERICHTETE EVOLUTION
ÉVOLUTION DIRIGÉE ASSISTÉE PAR MARQUEUR

(30) Priority: 27.11.2007 US 4562 P
(43) Date of publication of application: 15.09.2010
(73) Proprietor: VIB VZW, 9052 Gent (BE); Zabeau & Partners Bvba, 9000 Gent (BE)
(72) Inventor: ZABEAU, Marc, 9000 Gent (BE)
(86) International application number: PCT/EP2008/066319
(87) International publication number: WO 2009/068604

(56) References cited:
- EP-A- 0 967 291
- WO-A-2007/037678
- WO-A-2007/073165
- FOOLAD M R ET AL: "Identification and validation of QTLs for salt tolerance during vegetative growth in tomato by selective genotyping" GENOME, vol. 44, no. 3, June 2001 (2001-06), pages 444-454, XP002510761 ISSN: 0831-2796
- SARI-GORLA M ET AL: "Identification of genetic factors for alachlor tolerance in maize by molecular markers analysis" MOLECULAR AND GENERAL GENETICS, SPRINGER VERLAG, BERLIN, DE, vol. 251, no. 5, 1 January 1996 (1996-01-01), pages 551-555, XP002270593 ISSN: 0026-8925
- THOMPSON C J ET AL: "CLONING OF ANTIBIOTIC RESISTANCE AND NUTRITIONAL GENES IN STREPTOMYCETES" JOURNAL OF BACTERIOLOGY, vol. 151, no. 2, 1982, pages 668-677, XP002510986 ISSN: 0021-9193
- FEREA TRACY L ET AL: "Systematic changes in gene expression patterns following adaptive evolution in yeast" PROCEEDINGS OF THE NATIONAL ACADEMY OF SCIENCES OF THE UNITED STATES OF AMERICA, vol. 96, no. 17, 17 August 1999 (1999-08-17), pages 9721-9726, XP002510768 ISSN: 0027-8424
- MARULLO PHILIPPE ET AL: "Efficient use of DNA molecular markers to construct industrial yeast strains" FEMS YEAST RESEARCH, vol. 7, no. 8, 20 September 2007 (2007-09-20), pages 1295-1306, XP002510769 ISSN: 1567-1356 cited in the application
- GILL R T ET AL: "Genome-wide screening for trait conferring genes using DNA microarrays" PROCEEDINGS OF THE NATIONAL ACADEMY OF SCIENCES OF THE UNITED STATES OF AMERICA, vol. 99, no. 10, 14 May 2002 (2002-05-14), pages 7033-7038, XP002510760 ISSN: 0027-8424

## Description

The present invention is directed at a novel method to enhance the genetic improvement of industrial microorganisms. More specifically, the present invention is directed at molecular methods for the identification of genetic variants that contribute to the genetic improvement of the microorganisms. The methods of the present invention comprise novel approaches for identifying genetic markers that linked to said genetic variants, and for using said genetic markers to select improved industrial microorganisms. In one of the embodiments, the genetic markers linked to said genetic variants are identified in microbial populations that are subjected to directed evolution. In another embodiment the genetic markers linked to said genetic variants are identified in microbial sub populations that have been prescreened for improved characteristics. The primary benefits of the use of the methods of the present invention include the speeding up of the directed evolution process for the genetic improvement of industrial microorganisms and the enhancement of the improvement itself. Indeed the improved industrial microorganisms obtained with the methods of the present invention are superior over those obtained by conventional methods because the methods allow more beneficial genetic variants to be combined together in a single genetic improvement step and in a single organism. Finally, it is anticipated that the methods of the present invention can be utilized on any industrial microorganism for which natural genetic diversity is available, including bacteria, yeasts, fungi and algae.

Cells in culture, such as bacterial cells, yeast cells, fungal cells, algae, plant cells, insect cells or animal cells play an important role in biotechnology. Such cells are used in food production, for the production of primary and secondary metabolites, for enzyme production, for heterologous protein productions and many other applications.

A lot of efforts have been paid in strain improvement and directed evolution. This is mainly realized by iterations of random mutagenesis or recombination, followed by screening or selection. Although mutagenesis may be useful in bacteria, this approach is far more difficult in industrial *Saccharomyces* strains, which are often polyploidy or aneuploid. Moreover, the random mutagenesis will give rise to both positive and negative mutations, putting additional stress on the selection procedure which should be sensitive enough to select for rare events.

Recently, several DNA shuffling were developed to create libraries of chimeric genes by in vitro recombination. DNA shuffling is more efficient than random serial mutagenesis; whereas most of the random mutations are not functional, most variants formed by DNA shuffling are functional (Bacher et al, 2002). The main drawback however is that the method as such is only suitable for single gene traits, and that it is difficult if not impossible to apply the system to multigenic traits.

Delcardayre et al. (WO9831837; WO0004190) adapted the system of genomic shuffling for multigenic characteristics, by applying several rounds of recombination and selection. The method, in fact, is a variant of the serial mutagenesis, using now serial recombination for strain improvement. Although the method has certainly advantages compared with the random mutagenesis, the multiple rounds of recombination are time consuming and, depending on the number of genes that is involved in the determination of the desired characteristics, it is rather unlikely that one single strain in the population harbors the optimal combination of essential genes needed for a maximal expression of the studied characteristic. The selection of a genetically improved organism is thus likely to require iterative cycles of recombination and selection.

In plant and animal variety improvement, multigenic traits have often been improved by marker assisted breeding (Foolad et al., 2001 Varshney et al., 2005; Varshney et al., 2007, Abasht et al., 2006). However, in many cases, although there is a long history of industrial use, microbial strain improvement by breeding, especially improvement by marker assisted breeding has been of limited use. This is partly due to the unavailability - or limited availability of sexual crossing in most cases, but it can surely also be attributed to the absence of reliable markers that can be used for breeding of multigenic traits. Indeed, genetic markers such as Quantitative Trait Loci (QTL's) have not been described in bacteria, and only recently some QTL's in yeast were identified (Marullo et al., 2007; Nogami et al., 2007; Steinmetz et al, 2002), and used in yeast breeding (Marullo et al., 2007b). Determining those QTL's has been time consuming, and yeast QTL's for industrial traits are still missing.

The present invention provides a method for rapid identification of QTL's of industrial important traits in cells, which can be applied to all cells of which genetically mixed populations can be cultured in liquid culture. The QTL'S can be used for further breeding, either by sexual crossing of by direct cloning of the locus or loci into a suitable recipient strain.

A first aspect of the invention is a method for identifying one or more DNA polymorphisms linked a trait of interest in a micro-organism, comprising (i) selecting at least two parental strains, representing a significant polymorphism and/or differing in said trait of interest (ii) generating a population of descendants from said parental strains, whereby the members of the population comprise genetic material of at least two parental strains (iii) determining the ratio of the parental alleles in said population for at least one DNA polymorphism (iv) growing said population under conditions that are selective for said the trait of interest (v) determining the evolution of the ratio of said parental alleles during and/or at the end of said selective growth (vi) identifying at least one DNA polymorphism for which the ratio of said alleles evolves during/and or at the end of said selective growth. One preferred embodiment is the method whereby said micro-organism as a bacterium. Another preferred embodiment is the method whereby said micro-organism is a yeast. Preferably said yeast is a *Saccharomyces* sp. Parental strains can be all strains differing sufficiently either phenotypically or genotypically form each other. Sufficiently, as used here, means that the differences can be measured, resulting in a significant difference, when using the techniques known to the person skilled in the art. In the simplest embodiment, two haploid strains are sexually crossed to form a diploid, and then segregation is induced to obtain a pool of haploids, whereby the parental genes are distributed between the descendants. However, several methods can be used to increase the genetic diversity of the initial population. As a non limiting example, the parental strains can be diploid, and the segregating haploids can be used in mass mating to create a new pool of diploids, giving a complex pool of genetically diverse haploids upon segregation. Alternatively, during the crossing and segregation process, preferably at the level of the pool of haploids, the strain, or mixture of strains is transformed with a genetic library, coming from a third parental strain. In a preferred embodiment the strains used for crossing or as host for transformation do have a hyperrecombinant phenotype, facilitating mitotic and/or meiotic recombination. Genes involved in mitotic and/or meiotic recombination are known to the person skilled in the art. Preferably such gene is a gene that needs to be overexpressed to obtain the hyperrecombination phenotype; even more preferably said gene is located on a plasmid. In the latter case, plasmid loss can be induced at the end of the selective growth, to obtain a stable strain.

Another embodiment is a genomic shuffling experiment, using protoplast fusion, as described in WO0004190. In that case, after one of the successive recombination steps, no selection procedure is carried out, but the genetically diverse pool is cultivated. Such a genomic shuffling can be combined with classic sexual crossing and segregation. Determining the ratio of the parental alleles can be by any method known to the person skilled in the art. As a non-limiting example, depending upon the number of polymorphisms one wants to analyze, quantitative PCR can be used, or quantitative hybridization such as macro- or microarrays. Preferably, all polymorphism analyzed, even more preferably, those polymorphism are analyzed by sequence analysis.

The parental organisms are selected in such a way that they mildly or strongly differ in the trait of interest. Growth conditions are chosen in function of said trait of interest for which one wants to determine linked polymorphisms. Preferably, the growth conditions are selected in such a way that one of the parental organisms cannot grow under the conditions used. However, more stringent conditions can be used too, to obtain a better selection. More stringent conditions as used here means that the selection pressure with respect to the trait of interest is higher than for less stringent conditions. Alternatively, the growth conditions are adapted from less stringent to more stringent during the growth. Preferably selective growth conditions are applied in repeated batch, even more preferably selective growth conditions are applied in continuous culture. In case of a continuous culture, the dilution rate is adapted to the growth rate of the faster growing strains, so that slower growing strains are washed out of the culture. Applying repeated batch or continuous culture at high dilution rate has the advantage that the changes in the ratio of the relevant parental alleles will be more pronounced. Apart from the determination of the ration at time 0 of the culture, the ratio can be determined during and/or at the end of the culture; intermediate values can help to increase the significance in case of slow shifts, or they may give an indication upon possible interdependence of genes needed for a certain trait of interest. Indeed, it is possible that one allele needs to be present before another allele can become functional in respect to the trait of interest. A shift in alleles identifies a QTL. It indicates that the polymorphism identified is linked to a locus that contributes to the trait of interest, however without necessitating however that the polymorphism is located within a gene that is directly involved in the trait of interest.

The method of the present invention is thus well suited to the rapid and comprehensive identification of genetic markers linked to QTL's that contribute to the growth under the selective conditions. The person skilled in the art will realize that these genetic markers may be used to identify those individuals in a population that are best adapted to the selective conditions. Indeed, those individuals in the population that harbor the largest number of the genetic markers are candidates for being best adapted. Hence one does not have to await the outcome of the directed evolution experiment to obtain the best adapted strains. Moreover, it should be realized that the best adapted strain, namely the strain with a genotype that comprised all the optimal alleles, is either not present in the population, or in very low frequencies. Hence the selection of the best adapted strain may require iterative selection using the genetic markers identified on different populations. It is important to realize that in this case the best adapted strain can simply not be obtained by conventional directed evolution.

### EXAMPLES

### Example 1: Generating a segregating population of yeast

One diploid baker's yeast strain and one polyploidy wine yeast strain are selected on the base of their difference in resistance to ethanol. Those strains are grown on presporulation medium and then sporulation is induced on sporulation medium (1% potassium acetate, 2% agar). Spores are isolated, and in the diploid monosporic clones, resulting from the wine strain, the HO gene is inactivated by inserting a *ho::-KanMX4* cassette, as described by Marullo et al. (2006). The resulting strain is sporulated, and both an a and alpha heterothallic spore is crossed with the opposite mating type from the baker's yeast spores, to form two diploid strains. Said strains are again mass sporulated, and a and alpha segregants are isolated. The strains are pooled according to mating type, and used for growth under selective conditions

### Example 2: Growth under selective conditions

The pooled a and alpha yeast are grown in 10ml YPD, till saturation. The culture is used to pitch a 3 liter fermentor, at a concentration of 10⁶ cells/ml, in a medium of YPD with 10% ethanol. At time 0, a sample is taken for analysis of the allele ratio. Growth is anaerobic, at 28°C

At a density of 10⁸ cells/ml, a continuous regime is started, at a dilution of 0.2. Yeast growth is followed, and the ethanol concentration in the feed is slowly increased till no further increase in cell density is noted. On this point, a sample is taken for analysis. From that moment, both dilution rate and ethanol concentration are kept at the same level, unless a further increase in cell density is noted. After full stabilization of the culture, i.e. no change in cell density for a period of 24 hours, a new sample is taken for analysis

### Example 3: sequence analysis of the samples and identification of the QTL's.

DNA is isolated from each sample, digested with a combination of two restriction enzymes and size fractionated on agarose gels. About 1000 different restriction fragments are obtained. Each sample is ligated to barcode 454 adaptors, mixed in equimolar amounts and is submitted to sequencing.

One full plate 454 is performed on the mix of the samples, according to the procedure of Roche.

The different restriction fragment sequences are analyzed and their position on the genome is determined. Only those restriction fragments giving a representative number of sequences per sample are used for further computational analysis. For each sample, the ratio of the alleles is calculated for each restriction fragment, and this ratio is compared to the starting ratio in the segregating population. In case the locus is neutral, the ration of the alleles fluctuates but remains essentially unchanged. For loci under selection, the ratio of the alleles exhibits a gradual change consistent with the duration of the selection. Restriction fragments that give the highest ration shift comprise the most tightly genetically linked markers.

### Example 4: Construction of the final strain

The presence of the relevant QTL's is analyzed in the starting population of a and alpha cells. Suitable a and alpha strains, incorporating complementary QTL's are crossed, to obtain a diploid comprising all parental relevant QTL's. The resulting diploid is sporulated, spores are analyzed and the haploids are backcrossed; the procedure is repeated till all relevant QTL's are incorporated in one diploid strain. This strain is tested for ethanol production and ethanol tolerance, and compared with the parental wine strain. A significant increase both in ethanol production rate as well as in ethanol tolerance is found.

### REFERENCES

- Abasht, B., Dekkers, J.C. and Lamont, S.J. (2006). Review of quantitative trait loci identified in the chicken. Poult. Sci. 85, 2079-2096.
- Bacher, J.M., Reiss, B.D. and Ellington, A.D. (2002). Anticipatory evolution and DNA shuffling. Genome Biology, 3, 1021.1-1021.4.
- Foolad, M.R., Zhang, L.P. and Lin G.Y (2001). Identification and validation of QTLs for salt tolerance during vegetative growth in tomato by selective genotyping. Genome 44, 444-454.
- Marullo, P, Bely, M., Masneuf-Pomarède, I., Pos, M., Aigle, M and Dubourdieu, D. (2006). Breeding strategies for combining fermentative qualities and reducing off-flavor production in a wine yeast model. FEMS yeast res. 6, 268-279.
- Marullo, P., Aigle, M., Bely, M., Masneuf-Pomarède, I., Durrens, P., Dubourdieu, D and Yvert, G. (2007a). Single QTL mapping and nucleotide-level resolution of a physiological trait in wine Saccharomyces cerevisiae strains. FEMS Yeast Res., 7, 941-952.
- Marullo, P., Yvert, G., Bely, M., Aigle, M. and Dubourdieu, D. (2007b). Efficient use of DNA molecular markers to construct industrial yeast strains FEMS Yeast Res., 7, 1295-1306.
- Nogami, S., Ohya, Y and Yvert, G. (2007). Genetic complexity and Quantitative Trait Loci mapping of yeast morphological traits. PLoS Genetics, 3, 305-318
- Steinmetz, L.M., Sinha, H., Richards, D.R., Spiegelman, J.I, Oefner, P.J., McCusker, J.H. and Davis, R.W. (2002). Dissecting the architecture of a quantitative trait locus in yeast. Nature, 416, 326-330.
- Varshney, R.K., Graner, A. and Sorrells, M.E. (2005). Genomics-assisted breeding for crop improvement. Trends Plant Sci. 10, 621-630.
- Varshney, R.K., Langridge, P. and Graner, A. (2007). Application of genomics to molecular breeding of wheat and barley. Adv. Genet. 58, 121-155.

## Claims

1. A method for identifying one or more DNA polymorphisms linked to a trait of interest in a micro-organism, comprising (i) selecting at least two parental strains, representing a significant polymorphism and/or differing in said trait of interest (ii) generating a population of descendants from said parental strains and pooling said population, whereby the members of the pooled population comprise genetic material of at least two parental strains (iii) determining the ratio of the parental alleles in said population for at least one DNA polymorphism on a nucleic acid sample prepared from a sample of the pooled population (iv) growing said pooled population in liquid culture under conditions that are selective for said trait of interest (v) determining the evolution of the ratio of said parental alleles during and/or at the end of said selective growth on a nucleic acid sample prepared from a sample of the pooled population (vi) identifying at least one DNA polymorphism for which the ratio of said alleles evolves during/and or at the end of said selective growth.

2. The method according to claim 1, whereby said micro-organism is a bacterium.

3. The method according to claim 1, whereby said micro-organism is a yeast.

4. The method according to claim 3, whereby said yeast is *Saccharomyces cerevisiae.*

5. The method according to any of the preceding claims, whereby said determination of the ratio is carried out by sequencing.

## Patentansprüche

1. Verfahren zur Identifizierung eines oder mehrerer DNA-Polymorphismen, der bzw.
die mit einem Merkmal von Interesse in einem Mikroorganismus verknüpft ist bzw. sind, wobei das Verfahren Folgendes umfasst: (i) Auswählen von mindestens zwei Elternstämmen, die einen wesentlichen Polymorphismus darstellen und/oder sich in Bezug auf das Merkmal von Interesse unterscheiden, (ii) Erzeugen einer Population von Abkömmlingen der Elternstämme und Poolen der Population, wobei die Mitglieder der gepoolten Population genetisches Material von mindestens zwei Elternstämmen umfassen, (iii) Bestimmen des Anteils der Elternallele in der Population für mindestens einen DNA-Polymorphismus auf einer Nukleinsäureprobe, die aus einer Probe der gepoolten Population hergestellt wurde, (iv) Züchten der gepoolten Population in einer Flüssigkultur unter Bedingungen, die für das Merkmal von Interesse selektiv sind, (v) Bestimmen der Entwicklung des Anteils der Elternallele während und/oder zum Ende des selektiven Wachstums auf einer Nukleinsäureprobe, die aus einer Probe der gepoolten Population hergestellt wurde, (vi) Identifizieren mindestens eines DNA-Polymorphismus, für den der Anteil der Allele sich während und/oder zum Ende des selektiven Wachstums entwickelt.

2. Verfahren nach Anspruch 1, wobei der Mikroorganismus eine Bakterie ist.

3. Verfahren nach Anspruch 1, wobei der Mikroorganismus eine Hefe ist.

4. Verfahren nach Anspruch 3, wobei die Hefe *Saccharomyces cerevisiae* ist.

5. Verfahren nach einem der vorhergehenden Ansprüche, wobei die Bestimmung des Anteils durch Sequenzierung durchgeführt wird.

## Revendications

1. Procédé d'identification d'un ou plusieurs polymorphismes d'ADN liés à un trait d'intérêt dans un microorganisme, comprenant (i) la sélection d'au moins deux souches parentales représentant un polymorphisme important et/ou différant au niveau dudit trait d'intérêt, (ii) la génération d'une population de descendants à partir desdites souches parentales et le regroupement de ladite population, moyennant quoi les membres de la population regroupée comprennent du matériau génétique d'au moins deux souches parentales, (iii) la détermination du rapport des allèles parentaux dans ladite population pour au moins un polymorphisme d'ADN sur un échantillon d'acide nucléique préparé à partir d'un échantillon de la population regroupée, (iv) la croissance de ladite population regroupée dans une culture liquide dans des conditions qui sont sélectives pour ledit trait d'intérêt, (v) la détermination de l'évolution du rapport desdits allèles parentaux au cours de et/ou à la fin de ladite croissance sélective sur un échantillon d'acide nucléique préparé à partir d'un échantillon de la population regroupée, (vi) l'identification d'au moins un polymorphisme d'ADN pour lequel le rapport desdits allèles évolue au cours de et/ou à la fin de ladite croissance sélective.

2. Procédé selon la revendication 1, moyennant lequel ledit microorganisme est une bactérie.

3. Procédé selon la revendication 1, moyennant lequel ledit microorganisme est une levure.

4. Procédé selon la revendication 3, moyennant lequel ladite levure est *Saccharomyces cerevisiae.*

5. Procédé selon l'une quelconque des revendications précédentes, moyennant lequel ladite détermination du rapport est effectuée par séquençage.
